# EUROPÄISCHE PATENTSCHRIFT

(11) **EP 0 799 419 B1**
(45) Veröffentlichungstag und Bekanntmachung des Hinweises auf die Patenterteilung: **10.05.2000**
(21) Anmeldenummer: 95940155.5
(22) Anmeldetag: 06.12.1995
(51) Int. Cl.: G01N 33/36, D01G 23/06

(54) **VERFAHREN UND VORRICHTUNG ZUR KONTINUIERLICHEN MESSUNG DER MASSE EINES BEWEGTEN FASERBANDES**
PROCESS AND DEVICE FOR CONTINUOUSLY MEASURING THE MASS OF A MOVING FIBRE STRIP
PROCEDE ET DISPOSITIF PERMETTANT DE MESURER EN CONTINU LE POIDS D'UN RUBAN DE FIBRES MOBILE

(30) Priorität: 22.12.1994 DE 4445720
(43) Veröffentlichungstag der Anmeldung: 08.10.1997
(73) Patentinhaber: CSM-Sächsische Spinnereimaschinen GmbH, 09120 Chemnitz (DE)
(72) Erfinder: BÖHME, Dietmar, D-09130 Chemnitz (DE); HESSBERG, Silke, D-08144 Stenn (DE); KÄMPF, Lothar, D-09122 Chemnitz (DE); KÖPPLER, Horst, D-01558 Grossenhain (DE); MILLAUER, Olaf, D-09130 Chemnitz (DE); SIMON, Lothar, D-09111 Chemnitz (DE)
(74) Vertreter: Schneider, Manfred
(86) Internationale Anmeldenummer: DE9501758
(87) Internationale Veröffentlichungsnummer: WO9619728

(56) Entgegenhaltungen:
- WO-A-93/13407
- DE-A- 2 933 297
- DE-A- 3 635 267
- GB-A- 2 259 760
- SOVIET PATENT ABSTRACTS Section Ch, Week 8850 Derwent Publications Ltd., London, GB; Class F02, AN 88-359038 & SU,A,1 396 056 ( EXTRAMURAL TEXTILE) , 15.Mai 1988

## Beschreibung

Die Erfindung bezieht sich auf ein Verfahren und eine Vorrichtung zur kontinuierlichen Messung der Masse eines bewegten Faserbandes in einem das Faserband umschließenden Führungselement, wobei das Faserband mittels Strahler quer durchstrahlt wird, die das Faserband verlassende Reststrahlung mittels Meßelement, das nach einer durch physikalische Gesetze bestimmten Kennlinie arbeitet, gemessen wird, die gemessene Reststrahlung in elektrische Signale gewandelt wird und die elektrischen Signale einer elektronischen Verarbeitungseinheit für Anzeige-, Speicher- und / oder Stellprozesse zugeführt werden.

Ein Verfahren und eine Vorrichtung der beschriebenen Art ist durch die DD-PS 274 241 bekannt geworden.

Bei diesem Verfahren wird das Faserband durch einen an sich bekannten Trichter auf einen relativ kleinen Querschnitt komprimiert. Am Umfang dieses komprimierten Faserbandes wird auf einer Seite des Bandes ein Strahler angeordnet. Gegenüber befindet sich ein Meßelement in Form einer Photodiode, die die Strahlungsintensität auf der dem Strahler gegenüberliegenden Seite erfaßt und als elektrisches Signal der weiteren Verwertung zuführt.

Dieses so gewonnene Meßergebnis ist fehlerbehaftet, insbesondere dann, wenn Faserbänder unterschiedlicher Farbe und unterschiedlicher Reflexionseigenschaften gemessen werden.

Zur Ausschaltung dieses Fehlers des Meßsignals ist innerhalb der Faserbandführung eine zweite Meßstelle vorgesehen.
Die zweite Meßstelle ist durch eine V-förmige Anordnung von Strahler und Meßelement gekennzeichnet. Sie erfaßt im wesentlichen nur ein Maß für die reflektierten Strahlen.
Dieses Signal wird gemeinsam mit dem durch Transmission gewonnenen Signal erfaßt, verarbeitet und in ein Signal gewandelt, das der Masse des Faserbandes im jeweils gemessenen Bereich proportional sein soll.

Es hat sich jedoch gezeigt, daß dieses Verfahren zur Korrektur des Meßwertes mit Hilfe des Reflexionswertes aus dem Meßvorgang nicht geeignet ist, die Proportionalität zwischen Meßwert und tatsächlicher Masse des Faserbandes zu sichern.

Durch die DD 274 242 wird eine weitere Verfahrensweise zur Korrektur eines Meßsignales in Abhängigkeit von der Farbe und den Reflexionseigenschaften eines Faserbandes vorgeschlagen.
Bei diesem Verfahren wird das Faserband nacheinander in Bereichen gemessen, in denen es im unterschiedlichen Maße komprimiert ist.
Die unterschiedliche Größe der Transmissionsanteile bei unterschiedlicher Komprimierung dient als Ausgangswert für die Korrektur des Meßsignales.

Auch diese Verfahrensweise ist nicht geeignet, Meßwerte zu erzielen, die der Masse des gemessenen Faserbandes direkt proportional sind.

Mit der DE 41 06 567 A1 wird eine weitere Variante eines Meßverfahrens und einer entsprechenden Meßvorrichtung vorgeschlagen.

Bei diesem-Verfahren wird das Faserband ebenfalls in der bereits beschriebenen Weise im zu messenden Bereich verdichtet.
In der Meßebene sind mehrere, sich gegeseitig kreuzende Meßanordnungen, bestehend aus Strahler und Meßelement angeordnet.
Dabei sind jedem Meßelement zwei oder mehrere Strahler zugeordnet. Mit dieser Maßnahme wird offensichtlich das Ziel verfolgt, dem Meßelement ein ausreichend großes und stabiles Lichtsignal zuzuleiten, ohne den Strahler mit einer entsprechend großen Strahlungsleistung zu beaufschlagen.

Zwischen dem Strahler und der Oberfläche des Faserbandes und zwischen dem Faserband und dem Meßelement ist ein Glasring angeordnet, der ein ungestörtes Gleiten des Faserbandes gewährleisten soll und gleichzeitig als lichtdurchlässiges Element den Meßvorgang ermöglicht.

Es hat sich jedoch gezeigt, daß ein erheblicher Teil der durch den Strahler aufgebrachten Lichtleistung nicht das Faserband durchdringt, sondern im Ring weitergeleitet wird.

Die mit dieser Vorrichtung erzeugten Meßwerte sind in gleicher Weise fehlerbehaftet wie es bereits in Bezug auf die vorher genannten Literaturstellen dargestellt wurde.
An diesen fehlerhaften Meßergebnissen ändert auch die Anwendung des allgemein bekannten Impulsverfahrens nichts.

Durch die europäische Patentschrift 0 509 187 A1 wurde versucht, anstelle der Lichtstrahlung akustische Strahlen zur Messung der Bandmasse einzusetzen.

Ein Vibrator erzeugt Schwingungen, die das Band durchqueren und nach entsprechender Dämpfung durch das Band durch ein Mikrophon erfaßt und in elektrische Signale gewandelt werden.

Der Grad der Dämpfung der Schwingungen durch das Faserband sollte dabei der Masse des Faserbandes proportional sein.
Dieser Fall tritt in der Praxis nicht ein.
Der Meßwert wird insbesondere durch die Leitung der Schwingungen durch den Körper des Meßgehäuses so verfälscht, daß die Meßwerte im praktischen Betrieb nicht verwertbar sind.

Es ist die **Aufgabe der Erfindung**, die Meßwerte, unabhängig von der Farbe des Faserbandes und den Reflexionseigenschaften der Fasern, so zu gestalten, daß sie der tatsächlichen Masse des Faserbandes im jeweils gemessenen Bereich proportional sind.

Diese so gestellte Aufgabe wird durch das im Anspruch 1 definierte Verfahren auf einfache Weise und mit überraschenden Ergebnissen gelöst.

Das im Anspruch 1 definierte Verfahren basiert auf dem Grundgedanken, daß mit Hilfe der Transmissionsmessung stets ein Wert erfaßt wird, der - gleichbleibende Partie der Faserbänder vorrausgesetzt - der Masse des Faserbandes proportional ist.

Unterschiedliche Farben der Faserbänder und unterschiedliche Reflexionseigenschaften siedeln diesen Meßwert jedoch in unterschiedlichen Meßbereichen des Meßelementes an.

Die unterschiedliche Form der Kennlinie der Meßelemente führt dann dazu, daß gleiche Unterschiede in den erfaßten Strahlen zu unterschiedlichen elektrischen Signalen gewandelt werden.

Diese Signale sind in unterschiedlichen Meßbereichen unterschiedlich proportional zur Masse des gemessenen Faserbandes.

Mit dem vorliegenden Verfahren wird gewährleistet, daß durch die Anpassung der Strahlungsleistung an einen auf der Kennlinie des Meßelementes ausgewählten, optimalen elektrischen Mittelwert, dem Vorgabewert, stets im gleichen Meßbereich gearbeitet wird.
Die Meßwerte können dadurch anhand bekannter und genau erfaßbarer physikalischer Vorgänge genau definiert und gegebenenfalls exakt korrigiert werden.

Die Erfindung ermöglicht so eine hochgradig proportionale Gestaltung der Meßwerte, bezogen auf die Masse des jeweils zu messenden Faserbandabschnittes.

Wählt man den Vorgabewert und damit den optimalen Meßbereich nach Anspruch 2, kann man mit einfachen technischen Mitteln die Proportionalität zwischen der Masse des Faserbandes und dem gewonnenen Meßwert sichern.
Die Verwendung von Lichtstrahlen für die Messung der Banddicke gibt die Möglichkeit, äußere Störeinflüsse durch einfache Maßnahmen weitgehend auszuschalten.

Das Einstellen der Leistung des Strahlers nach Anspruch 4 sichert bei ausreichender Präzision der Strahlungsleistungen eine schnelle und zuverlässige Arbeitsweise unter Einsatz der, an der Maschine vorhandenen Steuerungen.

Mit der in Anspruch 5 definierten Vorrichtung läßt sich das eingangs genannte Verfahren problemlos realisieren.

Mit dem Begriff der "Umschalteinheit" wird hier ein Schaltelement bezeichnet, das geeignet ist, die Steuereinheit für die Leistung des Strahlers,
- entweder in Abhängigkeit von ausgewerteten Meßergebnissen durch ein bestimmtes Programm des Rechners
- oder zum Zeitpunkt des Einsteuerns einer neuen Partie von Faserstoffen oder Faserbändern in den Produktionsvorgang manuell ein- oder auszuschalten (Anspruch 6 und 7).

Durch die Zuordnung eines Pulsgebers nach Anspruch 8 wird gewährleistet, daß bei optimaler Strahlungsleistung für den Meßvorgang die insgesamt auf das Faserband aufgestrahlte Energie niedrig gehalten werden kann.

Anspruch 9 beinhaltet eine Anpassung der Meßvorrichtung an die Verfahrensweise nach Anspruch 3.

Die Gestaltung des Führungselementes nach Anspruch 10 erlaubt es, eine große Zahl von Meßstellen unter definierten Bedingungen über die gesamte Breite eines Faserbandes oder einer Schar von Faserbändern anzuordnen, deren Strahlung gemeinsam zu korrigieren und deren Meßergebnisse durch einfache Summierung zusammen zu fassen.

Das Führungselement und seine Einstellbarkeit nach Anspruch 11 gestattet es, an einer einzigen Meßanordnung das Messen von Faserbändern mit voneinander extrem abweichenden Masseverhältnissen durchzuführen.

Die Erfindung soll nachstehend an einem Ausführungsbeispiel näher erläutert werden. In den dazugehörigen Zeichnungen zeigen,

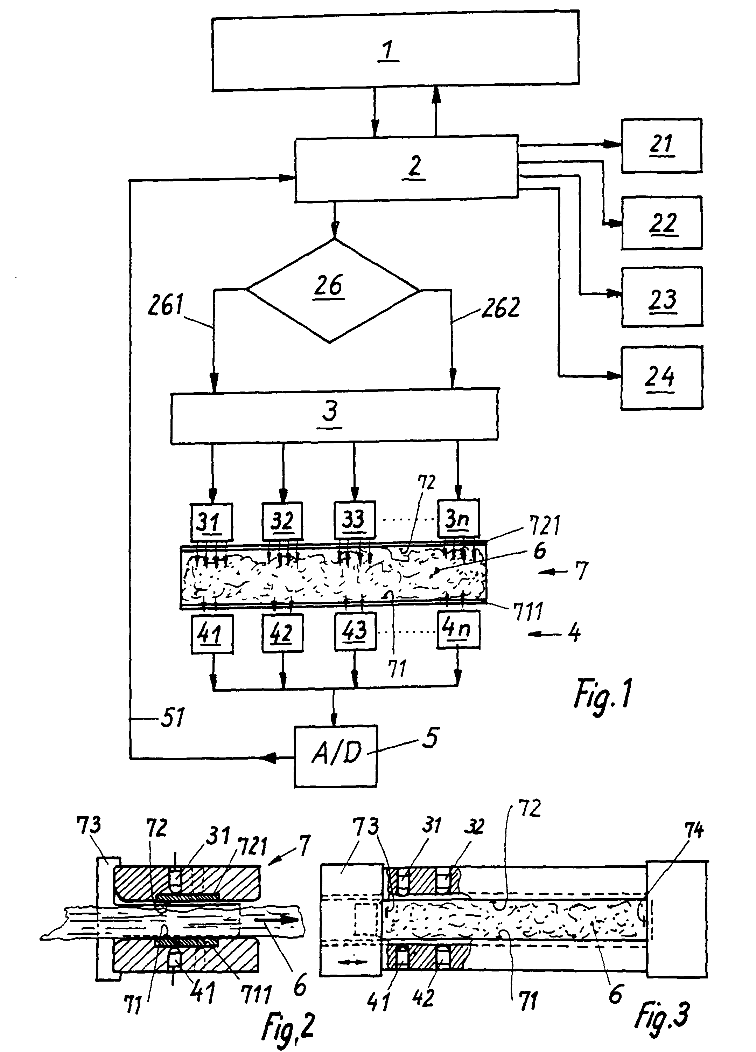

- Fig. 1: eine schematische Darstellung des Meßverfahrens in Form eines Blockschaltbildes,
- Fig. 2: einen Querschnitt durch die Meßvorrichtung längs der Bewegungsrichtung des Faserbandes und
- Fig. 3: eine Ansicht auf das Führungselement in Bewegungsrichtung des Faserbandes - teilweise geschnitten.

Zum Zwecke des Messens der Masse des Faserbandes 6 wird das Faserband 6 in einem quer zur Bewegungsrichtung allseitig begrenzten Führungselement 7 geführt. Für die Führung des Faserbandes 6 zum Zwecke der Messung der Masse wird lediglich die Forderung gestellt, daß die Grundfläche des Faserbandes 6 in der Ebene der Meßelemente 4 in etwa gleicher Dicke geführt wird. Das Faserband 6 wird vorzugsweise nicht komprimiert und muß die obere Führungsfläche 72 auch nicht berühren.

Innerhalb des Führungselementes 7 sind an der unteren Führungsfläche 71 und an der oberen Führungsfläche 72 Glasplatten 711, 721 vorgesehen.

Oberhalb der Glasplatte 711 befinden sich in einer oder mehreren Reihen in Bewegungsrichtung des Faserbandes hintereinander mehrere Strahler 31, 32, 33 ...3n.

Unterhalb der unteren Glasplatte 721 sind als Empfänger 4 ausgestaltete Meßelemente, z. B. Photodioden 41, 42, 43...4n eingefügt.

Den Strahlern 31, 32, 33 ...3n ist eine Steuereinheit 3 zugeordnet, die geeignet ist, den Strahlern 31, 32, 33 ...3n unterschiedliche Strahlungsenergie zuzuführen. Die Größe der zugeführten Strahlungsenergie wird durch eine symbolisch dargestellte Umschalteinheit 26 bestimmt. Sie führt entweder eine variable, korrigierte Strahlungsleistung über eine Leitung 261 oder eine konstante Strahlungsleistung - dargestellt durch eine ebenfalls symbolische Leitung 262 - zu.

Diese Umschalteinheit 26 ist wiederum einer Rechnereinheit 2 zugeordnet. Letztere ist in an sich bekannter Weise mit Datenspeichern 21, mit Speichern für die Qualitätsdatenerfassung 22, mit allgemeinen Auswerteeinheiten 23 (z.B. Reglern) und mit Anzeigeeinheiten 24 verbunden.
Dieser Rechnereinheit 2 kann eine weitere Steuerung 1 übergeordnet sein, die den Prozeß der kompletten Maschine oder einer ganzen Anlage steuert und kontrolliert.

Die Empfänger 4 erfassen die nach unten aus dem Faserband austretende Reststrahlung, die durch das Faserband nicht absorbiert wurde und wandeln diese entsprechend ihrer Kennlinie in elektrische Signale um.

Diese elektrischen Signale werden z. B. durch Addition zusammengefaßt und über einen A/D-Wandler 5 und die Leitung 51 für Auswertevorgänge der Rechnereinheit 2 zugeleitet.

Zum Zwecke der sogenannten Kalibrierung der Meßvorrichtung wird in das Führungselement 7 ein für die nachfolgend zu bearbeitende Partie von Faserbändern charakteristisches Faserband 6 eingelegt.

Zur Definition des gewünschten Meßbereiches bestimmt man anhand der Kennlinie der Meßelemente den optimalen Bereich, der für den Meßvorgang ausgewählt wird. Etwa in der Mitte dieses gewählten Meßbereiches legt man den sogenannten Vorgabewert fest und gibt diesen Wert der Rechnereinheit 2 als anzusteuernden Sollwert vor.
Jetzt stellt man die Umschalteinheit 26 auf die Vorgabe einer variablen Strahlungsleistung um.

Durch eine inkrementale Veränderung der Strahlungsleistung wird dieselbe beim Vorliegen eines Differenzsignales so an einen Wert herangeführt, bei dem das auf der Grundlage der gemessenen Reststrahlung ermittelte elektrische Signal in der Größe entsteht, die dem gewählten, mittleren Vorgabewert entspricht.

Auf diese Weise ist es möglich den Meßvorgang stets im optimalen Meßbereich durchzuführen.

Verändert sich im Laufe der Produktion aus irgendwelchen Gründen die Masse oder die Farbe des Faserbandes oder die Reflexionseigenschaften der Fasern so, daß die durchschnittlich gemessenen Werte um einen größeren Betrag ständig in gleicher Richtung vom Vorgabewert abweichen, kann die Rechnereinheit 2 oder 1 auf der Grundlage dieser Differenz die Umschalteinheit 26 betätigen und die Strahlungsleistung der Strahler 31, 32, 33 ...3n so nach oben oder unten korrigieren, daß die Meßwerte wieder gleichmäßig um den Vorgabewert plaziert sind.

Diesen Vorgang kann man als sogenannte dynamische Kalibrierung bezeichnen.
Diese dynamische Kalibrierung kann man auch gezielt dazu nutzen, die Meßvorrichtung nach der statischen Kalibrierung (Anpassung der Strahlungsleistung im Stillstand) auf einen auch dynamisch bestätigten Vorgabewert einzustellen.

Als strahlendes Medium kann man unterschiedliche Medien nutzen. Vorteilhaft sind Strahler im Bereich der Wellenlänge des Lichtes, weil diese Strahlen durch übliche Werkstoffe sicher abgeschirmt werden können.

Unter dem Begiff **kontinuierliche Messung** wird nicht nur eine solche Messung verstanden, bei der ununterbrochen ein Meßsignal anliegt.
Wir verstehen darunter auch eine solche Messung, bei der in einem ablaufenden Prozeß im erforderlichen Umfang notwendige Messungen durchgeführt werden, deren zeitlicher Abstand durch den Charakter des Prozesses ansich bestimmt wird.

### Aufstellung der verwendeten Bezugszeichen

- 1: Steuerung, übergeordnet

- 2: Rechnereinheit
- 21: - Datenspeicher
- 22: - Qualitätsdatenerfassung
- 23: - Auswertung, allgemein
- 24: - Anzeige
- 26: - Umschalteinheit
- 261: - - Leitung, variable Strahlungsleistung
- 262: - - Leitung, konst. Strahlungsleistung

- 3: Steuereinheit
- 31: - Strahler
- 32: - Strahler
- 33: - Strahler
- 3 n: - Strahler

- 4: Empfänger
- 41: - Meßelement, z.B.Photodiode
- 42: - Meßelement, z.B.Photodiode
- 43: - Meßelement, z.B.Photodiode
- 4 n: - Meßelement, z.B.Photodiode

- 5: A/D Wandler
- 51: Leitung
- 6: Faserband

- 7: Führungselement
- 71: - Führungsfläche
- 711: - - Glasplatte
- 72: - Führungsfläche
- 721: - - Glasplatte
- 731: - Führungsfläche, verstellbar
- 74: - Führungsfläche, fest

## Patentansprüche

1. Verfahren zur kontinuierlichen Messung der Masse eines bewegten Faserbandes in einem das Faserband umschließenden Führungselement, wobei
- das Faserband mittels Strahler quer durchstrahlt wird,
- die das Faserband verlassende Reststrahlung mittels Meßelement, das nach einer durch physikalische Gesetze bestimmten Kennlinie arbeitet, gemessen wird,
- die gemessene Reststrahlung in elektrische Signale gewandelt wird und
- die elektrischen Signale einer elektronischen Verarbeitungseinheit für Anzeige-, Speicher- und / oder Stellprozesse zugeführt werden,
dadurch gekennzeichnet,
daß die Intensität des Strahlers
- für eine Partie von Faserbändern
- bei Vorlage eines die Partie repräsentierenden Faserbandes im Führungselement
- in Abhängigkeit von einem vorgewählten, mittleren Vorgabewert eingestellt wird, und
daß der mittlere Vorgabewert ein elektrischer Wert ist,
- der sich etwa in der Mitte eines definierten Meßbereiches der Kennlinie des Meßelementes befindet.

2. Verfahren nach Anspruch 1, dadurch gekennzeichnet,
daß der mittlere Vorgabewert aus einem nahezu linearen und damit optimalen Meßbereich der Kennlinie des Meßelementes ausgewählt wird.

3. Verfahren nach einem der Ansprüche 1 oder 2,
dadurch gekennzeichnet,
daß der Strahler Lichtstrahlen, vorzugsweise aus dem Spektralbereich der Infrarotstrahlen sendet und
daß die Kennlinie des Meßgerätes die Kennlinie eines Phototransistors ist.

4. Verfahren nach Anspruch 1 bis 3, dadurch gekennzeichnet,
daß das Einstellen der Leistung des Strahlers bei Feststellung einer zulässigen Differenz zwischen dem Meßwert und dem mittleren Vorgabewert schrittweise bis zur Übereinstimmung des Meßwertes mit dem mittleren Vorgabewert erfolgt.

5. Vorrichtung zur kontinuierlichen Messung der Masse eines bewegten Faserbandes in einem das Faserband umschließenden Führungselement, nach dem Verfahren nach einem der Ansprüche 1 bis 4, bestehend
- aus einem Strahler (31,32,...3 n) und einem als Meßelement (41,42,....,4n) ausgebildeten Empfänger (4) an der Peripherie des Faserbandes (6),
- aus einem Wandler(41,42,...4 n) für die Wandlung der empfangenen Reststrahlen in elektrische Meßwerte und
- aus einer Recheneinheit (2) zur Verarbeitung der elektrischen Meßwerte,
**dadurch gekennzeichnet,**
daß dem Strahler (31,32,....3 n) eine Steuereinheit (3) für die Intensität des Strahlers (31,32,...3 n) zugeordnet ist und
daß der Steuereinheit (3) eine Umschalteinheit (26) vorgeordnet ist, die wahlweise eine konstante oder eine incrementierte Strahlungsleistung zuführt und
daß das Meßelement(4) in Abhängigkeit von den optischen Eigenschaften der zu verarbeitenden Partie von Faserbändern etwa in der Mitte eines definierten Meßbereiches seiner Kennlinie arbeitet.

6. Vorrichtung nach Anspruch 5, dadurch gekennzeichnet,
daß die Umschalteinheit (26) Bestandteil der Rechnereinheit (2) und ihres Programmes ist.

7. Vorrichtung nach Anspruch 5, dadurch gekennzeichnet,
daß die Umschalteinheit (26) aus einem handbetätigten Schalter besteht.

8. Vorrichtung nach einem der Ansprüche 5 bis 7, dadurch gekennzeichnet,
daß dem Strahler (31,32,...3 n) und/oder dem Empfänger (4) ein Pulsgeber zugeordnet ist.

9. Vorrichtung nach einem der Ansprüche 5 bis 8, dadurch gekennzeichnet,
daß der Strahler (31,32,...3 n) ein Lichtstrahler ist und der Empfänger (4, 41,42,...4 n) als Photodiode ausgebildet ist.

10. Vorrichtung nach einem der Ansprüche 5 bis 9, dadurch gekennzeichnet,
daß das, das Faserband (6) umschließende Führungselement (7) mindestens zwei zueinander parallele Führungsflächen 71, 72 für das Faserband (6) besitzt
und daß die Strahler (31,....3 n) und /oder die Empfänger (4, 41,...4 n) in diese Führungsflächen (71, 72) eingefügt sind.

11. Vorrichtung nach einem der Ansprüche 5 bis 10, dadurch gekennzeichnet,
daß das Führungselement (7) zusätzlich zu den zueinander parallelen Führungsflächen (71, 72) zwei weitere Führungsflächen (73, 74) besitzt, von denen mindestens eine Führungsfläche (73) parallel zu den ersten Führungsflächen (71, 72) einstellbar ist.

## Claims

1. Method for continuously measuring the mass of a moving fibre strip in a guide component surrounding the fibre strip, in which
- the fibre strip is transversely irradiated by radiators,
- the residual radiation leaving the fibre strip is measured by a measuring component operating on a characteristic determined by physical laws,
- the measured residual radiation is converted into electric signals and
- the electric signals are taken to an electronic processing unit for display, storage and/or adjusting processes,
characterized in that
the intensity of the radiator is set
- for a batch of fibre strips
- on the presentation of a fibre strip representing the batch in the guide component
- depending on a preselected, mean default value, and
that said mean default value is an electric value
- located approximately at the centre of a defined measuring range of the characteristic of the measuring component.

2. Method according to Claim 1, characterized in that the mean default value is selected from a nearly linear and therefore optimum measuring range of the characteristic of the measuring component.

3. Method according to one of Claims 1 or 2,
characterized in that
the radiator sends out light rays, preferably from the spectral range of infrared rays, and that the characteristic of the measuring component is that of a phototransistor.

4. Method according to Claims 1 to 3, characterized in that
the radiator intensity is set in increments whenever a permissible difference between the measured value and the mean default value is detected until the measured value is consistent with the mean default value.

5. Device for continuously measuring the mass of a moving fibre strip in a guide component surrounding said fibre strip in accordance with the method defined in Claims 1 to 4, consisting of
- a radiator (31, 32, 33 ... 3 n) and a receiver (4) designed as measuring component (41, 42, ... 4 n) at the periphery of the fibre strip (6),
- a transducer (41, 42, ... , 4 n) for converting the residual radiation received into electric measured values and
- a computer unit (2) for processing said electric values,
characterized in that
the radiator (31, 32,33 ... 3 n) is associated with a control unit (3) for the intensity of the radiator (31.32, ... 3n),
and that a changeover unit (26) precedes the control unit (3) which alternatively feeds in
- a constant or
- an incremented radiation intensity, and
that the measuring component (4) operates approximately at the median of its characteristic depending on the optical properties of the batch of fibre strips to be processed.

6. Device according to Claim 5, characterized in that the changeover unit (26) is an integral part of the computer unit (2) and its software program.

7. Device according to Claim 5, characterized in that the changeover unit (26) is a manually operated switch.

8. Device according to any one of Claims 5 to 7, characterized in that
the radiator (31, 32,... 3 n) and/or the receiver (4) is associated with a pulse generator.

9. Device according to any one of Claims 5 to 8, characterized in that
the radiator (31, 32,... 3 n) is a light radiator, and the receiver (4, 41, 42, ... 4 n is designed as a photodiode.

10. Device according to any one of Claims 5 to 9, characterized in that
the guide component (7) surrounding the fibre strip (6) comprises a minimum of two parallel guide surfaces (71, 72) for the fibre strip (6),
and that the radiators (31, ... 3 n) and/or receivers (4, 41, 4 n) are incorporated in said guide surfaces (71, 72).

11. Device according to any one of Claims 5 to 10, characterized in that
the guide component (7) comprises two more guide surfaces (73, 74) in addition to being parallel to each other guide surfaces (71, 72) of which at least one guide surface (73) can be adjusted in parallel to the first guide surfaces (71, 72).

## Revendications

1. Procédé pour la mesure en continu de la masse d'un ruban à fibres avancé dans un élément de guidage enserrant le ruban à fibres, lors de laquelle :
- le ruban à fibres est traversé par des rayonnements dans un sens transversal au moyen d'une source de rayonnement
- le rayonnement rétroréfringé quittant le ruban à fibres est mesuré par un élément de mesurage travaillant selon une caractéristique déterminée par des lois physiques
- le rayonnement rétroréfringé mesuré est converti en signaux électriques
- les signaux électriques sont alimentés par une unité de traitement électronique permettant des procès de visualisation, d'enregistrement et / ou de commande
et caractérisé par :
l'intensité d'une source de rayonnement
- réglée pour un lot de rubans à fibres
- lors de la présence dans l'élément de guidage d'un ruban à fibres représentant le lot
- en fonction d'un objectif d'une valeur moyenne présélectionnée
une valeur électrique de l'objectif de la valeur moyenne :
- qui se trouve plus ou moins au centre d'une gamme de mesures définie par la caractéristique de l'élément de mesure.

2. Procédé suivant revendication 1, caractérisé par :
le choix de l'objectif de la valeur moyenne sur une gamme de mesures linéaire, et par conséquent optimale, de la caractéristique de l'élément de mesure.

3. Procédé suivant une des revendications 1 ou 2, caractérisé par :
l'émission de rayons lumineux par la source de rayonnement, de préférence du domaine du spectre de l'infrarouge, la caractéristique de l'appareil de mesure étant la caractéristique d'un phototransistor.

4. Procédé suivant revendications 1 à 3, caractérisé par :
le réglage pas à pas de la capacité de la source de rayonnement, où une différence admissible entre la valeur mesurée et l'objectif de valeur moyenne est déterminée, jusqu'à ce que la valeur mesurée soit conforme à l'objectif de valeur moyenne.

5. Dispositif pour la mesure en continu de la masse d'un ruban à fibres avancé dans un élément de guidage enserrant le ruban à fibres, selon le procédé suivant une des revendications 1 à 4, composé de :
- une source de rayonnement (31, 32, ... 3n) et un récepteur (4) formé comme élément de mesure (41, 42, .... 4n) prévus à la périphérie du ruban à fibres (6)
- un convertisseur (41, 42, ....4n) pour convertir le rayonnement rétroréfringé reçu en valeurs électriques de mesure
- une unité de traitement de données (2) pour le traitement des valeurs électriques de mesure
et caractérisé par :
une unité de commande (3) pour l'intensité de la source de rayonnement ( 31, 32, ... 3n) associée à la source de rayonnement (31, 32, ... 3n),
une unité de commutation (26) prévue à l'amont de l'unité de commande (3) alimentant cette dernière au choix d'une puissance de rayonnement constante ou incrémentée,
le travail de l'élément de mesures (4) plus ou moins au milieu d'une gamme de mesures définie à partir de sa caractéristique, en fonction du lot de rubans à fibres à traiter.

6. Dispositif suivant revendication 5, caractérisé par :
l'unité de commutation (26) en partie intégrante de l'unité de traitement de données (2) et de son programme.

7. Dispositif suivant revendication 5, caractérisé par :
l'unité de commutation (26) consistant en un commutateur à commande manuelle.

8. Dispositif suivant une des revendications 5 à 7, caractérisé par :
un émetteur d'impulsions associé à la source de rayonnement (31, 32, ... 3n) et / ou au récepteur (4).

9. Dispositif suivant une des revendications 5 à 8, caractérisé par :
la source de rayonnement (31, 32, ... 3n) émettrice de lumière et le récepteur (4, 41, 42,... 4n) formé comme photodiode.

10. Dispositif suivant une des revendications 5 à 9, caractérisé par :
l'élément de guidage (7) enserrant le ruban à fibres (6) qui possède au moins deux surfaces de guidage 71, 72 parallèles l'une par rapport à l'autre pour le ruban à fibres (6),
l'encastrement des sources de rayonnement (31, ... 3n) et / ou des récepteurs (4, 41, ... 4n) dans lesdites surfaces de guidage (71, 72).

11. Dispositif suivant une des revendications 5 à 10, caractérisé par :
en addition des surfaces de guidage parallèles (71, 72), l'élément de guidage (7) qui possède deux autres surfaces de guidage (73, 74) dont au moins une surface de guidage (73) est réglable parallèlement aux premières surfaces de guidage (71, 72).
